(19) Europäisches Patentamt
European Patent Office
Office européen des brevets

(11) Numéro de publication: **0 121 468**
**B1**

(12) # FASCICULE DE BREVET EUROPÉEN

(45) Date de publication du fascicule du brevet: **03.02.88**

(51) Int. Cl.⁴: **A 61 K 37/02**, B 01 J 47/00, B 01 J 39/16

(21) Numéro de dépôt: **84400602.3**

(22) Date de dépôt: **26.03.84**

(54) **Procédé de fractionnement du plasma.**

(30) Priorité: **01.04.83 FR 8305441**

(43) Date de publication de la demande: **10.10.84 Bulletin 84/41**

(45) Mention de la délivrance du brevet: **03.02.88 Bulletin 88/5**

(84) Etats contractants désignés: **AT BE CH DE FR GB IT LI LU NL SE**

(56) Documents cité:
**DE-A-2 348 806**
**FR-A-2 319 399**
**FR-A-2 327 256**
**FR-A-2 403 098**
**US-A-4 025 500**
**US-A-4 136 094**
**US-A-4 228 154**

(73) Titulaire: **RHONE- POULENC RECHERCHES, 25 Quai Paul Doumer, F-92408 Courbevoie Cedex (FR)**
Titulaire: **INSTITUT MERIEUX Société anonyme dite:, 17, rue Bourgelat, F-69002 LYON (FR)**

(72) Inventeur: **Dromard, Adrien, 17, rue des Cerisiers, F-92700 Colombes (FR)**
Inventeur: **Exertier, Michel, 3, Rue Christine, F-91400 Orsay (FR)**
Inventeur: **Rollin, Claude, 13, Rue de Bretagne - Villabe, F-91100 Corbeil (FR)**
Inventeur: **Tayot, Jean- Louis, 1, Rue des Greffières, F-69890 La Tour de Salvagny (FR)**
Inventeur: **Tardy, Michel, 165 bis, Rue Joliot Curie, F-69005 Lyon (FR)**

(74) Mandataire: **Tavernier, Colette, RHONE- POULENC INTERSERVICES Service Brevets Chimie 25, Quai Paul Doumer, F-92408 Courbevoie Cédex (FR)**

**EP 0 121 468 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention a pour objet un procédé de fractionnement du plasma au moyen d'échangeurs d'ions, anionique et cationique, caractérisé en ce qu'une solution de plasma est mise en contact avec au moins un support partiellement hydrophobe, échangeur d'ions ou non, et au moins un support hydrophile échangeur d'ions, les supports étant choisis indépendamment dans le groupe des matrices de polymères naturels polysaccharidiques, des matrices de polymères synthétiques, des oxydes minéraux revêtus d'un polymère polysaccharidique et des oxydes minéraux revêtus d'un film de polymère synthétique réticulé.

La méthode la plus largement répandue à l'échelle industrielle pour séparer les principaux composants du plasma, albumine et immunoglobulines, est la méthode de Cohn dont le principe est basé sur la précipitation sélective des protéines à l'aide de solutions hydroalcooliques. Les procédés de précipitation ne sont pas parfaitement sélectifs et sont partiellement dénaturants; ils impliquent des manipulations longues et coûteuses en main d'oeuvre et en investissement, rendant l'obtention de produits stériles et apyrogènes particulièrement délicate.

Avec l'introduction des matériaux échangeurs d'ions, de nouvelles techniques ont été proposées pour séparer les protéines de leurs mélanges par des procédés en continu sur colonnes à lit fixe. Ces procédés sont plus économiques et permettent d'obtenir avec de meilleurs rendements des protéines de plus grande pureté. Cependant leur développement dans l'industrie biologique à grande échelle est encore limité par certains inconvénients.

Des procédés pour isoler l'albumine du plasma sanguin par contact avec des échangeurs d'ions polysaccharidiques ont été décrits dans les brevets FR-A-2 327 256, US-A-4 228 154 et US-A-4 136 094. Ces procédés impliquent des traitements préliminaires complexes pour éliminer la majeure partie des impuretés, lipoprotéines notament et, dans certains cas, des $\gamma$ globulines et/ou nécessitent une purification ultérieure de l'albumine par tamisage moléculaire pour obtenir un taux de pureté supérieur à 98 %. Ces traitements complémentaires sont difficilement industrialisables et peu économiques. La productivité est faible et inadaptée au traitement d'un volume important de plasma dont le besoin est ressenti par la demande mondiale en protéines, sans cesse croissante.

Les supports minéraux ont, à l'inverse des polysaccharides, d'excellentes qualités physico-mécaniques permettant des débits de filtration très élevés, mais ils présentent des sites d'absorption non spécifiques pouvant occasionner, en fonction du pH et de la force ionique utilisable, une perte de rendement en protéines.

Des procédés de séparation de protéines par chromatographie sur des supports minéraux échangeurs d'ions ont été décrits notamment dans les brevets FR-A-2 321 932, FR-A-2 359 634 et FR-A-2 464 967. L'association des échangeurs d'ions décrits peut conduire, dans le cas du fractionnement du plasma, à des rendements insuffisants pour les degrés de pureté exigés par les utilisations thérapeutiques et ne permet pas d'obtenir de l'albumine de haute pureté quel que soit le plasma à fractionner.

Le but principal de l'invention est de fournir un procédé permettant de traiter des volumes importants de plasma et d'isoler, sans traitement complémentaire autre que la clarification initiale du plasma, des protéines de haute pureté avec un bon rendement.

Un but plus particulier est de fournir un procédé d'extraction à grande échelle d'albumine, pour usage thérapeutique à partir de plasma et quelle que soit l'origine du plasma.

Dans son aspect le plus général, le procédé selon l'invention consiste à fractionner le plasma au moyen d'échangeurs d'ions anionique et cationique et est caractérisé en ce qu'une solution de plasma est mise en contact avec au moins un support partiellement hydrophobe échangeur d'ions ou non, et au moins un support hydrophile échangeur d'ions.

Par support partiellement hydrophobe, on entend un support possédant des groupements hydrophiles et hydrophobes capable de former des complexes avec des solutés porteurs de groupements lipophiles. Ils sont généralement constitués d'un polymère hydrophobe ou porteurs de chaînes alkyles accessibles à un nombre de carbone égal ou supérieur à 3, linéaires ou ramifiées, saturées ou non saturées, ou de noyaux cycliques saturés ou non saturés. Un support hydrophile est à l'inverse un support qui ne possède pas de groupements hydrophobes, les sites de surface étant exclusivement hydrophiles. Ces supports seront définis plus spécifiquement ci-après.

Selon un aspect plus particulier de l'invention, ce procédé est caractérisé en ce qu'une solution de plasma partiellement dessalé est mise en contact avec successivement au moins un échangeur d'anions puis un échangeur de cations les échangeurs dont la capacité d'échange est inférieure à 2 m.eq/g étant constitués d'un support minéral poreux ayant une granulométrie de 4 µm à 5 mm, un diamètre de pores de 500 à 2.500 Å (50 à 250 nm), une surface spécifique de 5 à 150 $m^2/g$ revêtu d'une quantité inférieure à 15 $mg/m^2$ d'un film de polymère réticulé contenant ou portant des groupes amines ou sels d'ammonium quaternaire pour au moins l'un des échangeurs, et d'un film de polyvinyllactame réticulé portant des groupes acide carboxylique pour l'autre échangeur.

Dans cet aspect particulier de l'invention, les supports minéraux servant de base aux échangeurs d'ions sont représentés par les alumines et les silices. Les supports des échangeurs mis en oeuvre peuvent être de même nature ou de nature différente, et posséder les mêmes caractéristiques ou des caractéristiques différentes, à condition de rester dans les limites indiquées ci-avant.

Le support minéral mis en oeuvre possède de préférence un diamètre poreux de 600 à 1.500 Å (60 à 150 nm), une surface spécifique de 20 à 50 $m^2/g$ et une granulométrie de 50 µm à 1 mm.

La résine échangeuse d'anions est formée d'un polymère réticulé contenant ou portant des groupes fonctionnels: amines primaire, secondaire ou tertiaire, sels d'ammonium quaternaire de formules générales: $-NH_2$, -NHR, $-N(R)_2$, $-N^{(+)}(R)_3X^{(-)}$, dans lesquelles R identique ou différent représente un groupe alkyle ou hydroxyalkyle ayant 1 à 4 atomes de carbone et X un anion minéral ou organique tel que, par exemple, chlorure, sulfate, nitrate, phosphate, citrate.

Les groupes amines tertiaires, sels d'ammonium quaternaire font partie de la chaîne du polymère réticulé, ou sont fixés au polymère réticulé qui revêt toute la surface du support et donnent des échangeurs, dont la capacité d'échange est inférieure ou égale à 2 meq/g et de préférence comprise entre 0,15 et 1,2 meq/g et plus préférentiellement entre 0,15 et 0,70 meq/g.

Les polymères réticulés qui revêtent la surface des supports sont des produits en eux-mêmes connus, obtenus à partir de monomères, tels que les composés époxydiques, qui réticulent avec les polyamines comme catalyseurs; le formaldéhyde, qui réticule par poly-condensation avec les polyamines; les monomères vinyliques: vinylpyridine, styrène et dérivés, qui réticulent avec des monomères polyfonctionnels, comme les discrylates ou diméthacrylates de mono- ou poly- alkylène-glycols, le divinylbenzène, les vinyltrialcoxysilanes, les vinyl-trihalogénosilanes, le bis-méthylène acrylamide, en présence d'un initiateur ou de rayons ultraviolets.

Dans le cas où le polymère réticulé ne possède pas dans sa chaîne de groupements fonctionnels, il est nécessaire de le modifier. C'est le cas notamment des polymères réticulés à base de styrène et dérivés, d'acrylates et méthacrylates d'alkyle et d'acrylonitrile. Cette modification du polymère est effectuée suivant tous procédés connus.

Ces résines échangeuses d'anions sur support et leurs procédés de préparation ont été décrites dans les demandes de brevets publiées FR-A-2 321 932 et FR-A-2 464 967 auxquelles on peut se référer.

S'agissant de l'échangeur de cations, le support minéral poreux est revêtu d'un film de polyvinyllactame réticulé portant des groupes fonctionnels -COOH.

Les copolymères de vinyllactame qui revêtent le support sont des produits en eux-mêmes connus. Ils sont obtenus par copolymérisation de monomères de formule:

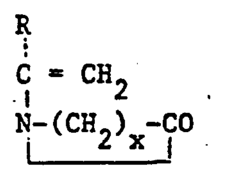
(I)

où R est H ou bas alkyl et x est un nombre entier compris entre 2 et 5, avec un monomère acide carboxylique insaturé de formule:

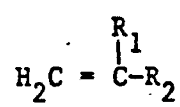
(II)

où $R_1$ est H ou $CH_3$ et $R_2$ est un groupe -COOH qui peut être relié à l'atome de carbone insaturé par l'intermédiaire d'un radical divalent alkylène linéaire ou ramifié en $C_1$-$C_{30}$, phénylène, cycloalkylène, $-(CH_2)_n-O-(CH_2)_m-$, $-(CH_2)_n-CO-(CH_2)_m-$, $-(CH_2)_n-COO-(CH_2)_m-$, $(CH_2)_n-SO_2-(CH_2)_m-$, $-(CH_2)_n-NR_4-(CH_2)_m-$, $(CH_2)_n-CO-NR_4-(CH_2)_m-$ où $R_4$ est H ou alkyle en $C_1$-$C_3$ et n, m est un nombre entier allant de 0 à 12.

Des exemples de monomàres de formule (I) sont la N-vinylpyrrolidone, le N-vinylcaprolactame, le N-vinyl β propiolactame, l'α méthylvinylpropiolactame. On utilise de préférence la N-vinylpyrrolidone.

Comme exemples d'acide carboxylique de fomule (II), on peut citer l'acide acrylique, l'acide méthacrylique, l'acide 4-pentenoïque, l'acide vinylbenzènecarboxylique, l'acide 6-acrylamidohexanoïque.

Ces monomères peuvent être réticulés à l'aide d'agents de réticulation polyfonctionnels connus dans la technique tels que par exemple, les diacrylates de polyols comme le diacrylate ou le diméthacrylate de diéthylène glycol ou de dibutanediol 1-4 et les dérivés vinyliques ou allyliques de triazine comme le triallylisocyanurate. Comme agent de réticulation, on peut encore mettre en oeuvre des monomères de type silanique de formule $CH_2=CH-Si\ X_3$ (III) dans laquelle chaque X, identique ou différent, représente un radical hydrolysable alcoxy inférieur, ou acetoxy ou phénoxy ou un halogène. Des exemples de dérivés silaniques disponibles sont le vinyltriméthoxysilane, le vinyltriéthoxysilane, le vinyltriacétoxysilane, le vinyltrichlorosilane, le vinyl tris (β methoxyéthoxy)silane.

De manière préférentielle, le revêtement du support minéral par le copolymère est réalisé par polymérisation in situ des monomères en présence du support. Le vinyllactame, le réticulant et l'acide vinylcarboxylique, éventuellement un initiateur, sont mis en solution dans un solvant, la solution est imprégnée sur le support puis le solvant est évaporé et les monomères réticulés par tout procédé connu en soi tels que chaleur ou rayonnements ultraviolets. Comme solvant, on met en oeuvre tous produits solvants des monomères dont le point d'ébullition est le plus bas possible pour favoriser son évaporation ultérieure. Ce sont, par exemple, le chlorure de méthylène, les chlorofluorométhanes (Flugènes), l'éther éthylique, l'acétone, l'acétate d'éthyle.

De manière générale, la quantité de monomères à mettre en oeuvre est choisie de manière à obtenir à la

surface des pores du support un film de polymère compris entre 1 et 15 mg/$m^2$, de préférence entre 1 et 6 mg/$m^2$. Le rapport des monomères détermine la quantité de groupements fonctionnels répartis sur le film polymère. De préférence, on utilise l'acide vinylcarboxylique en quantité telle que la quantité de groupes fonctionnels répartis à la surface du support soit comprise entre 0,05 et 2 m.eq/g de support. La quantité de réticulant peut varier entre 1 et 60 % en poids par rapport au poids total des monomères.

Poursuivant ses recherches, la demanderesse a trouvé que la séparation des protéines du plasma et l'obtention d'albumine de très haute pureté pouvaient être obtenues par chromatographie sur d'autres supports à condition de mettre en oeuvre successivement au moins un support partiellement hydrophobe échangeur d'ions ou non et au moins un support hydrophile échangeur d'ions.

Dans cet aspect général de l'invention, le support peut être minéral ou organique, naturel ou synthétique. Pour des raisons de productivité à grande échelle, on préfère toutefois les supports à base d'oxydes minéraux.

Les supports hydrophiles peuvent être constitués de matrices polymères ou d'oxydes minéraux revêtus d'un film de polymère hydrophile, porteurs de groupes échangeurs d'ions. Des exemples de supports naturels hydrophiles sont les matrices polymères à base de polysaccharides tels que le dextrane réticulé, l'agarose, l'agarose réticulé, la cellulose, la cellulose réticulée.

Des supports hydrophiles synthétiques connus peuvent être obtenus par polymérisation de monomères tels que l'acrylamide et ses dérivés hydrophiles.

Un autre groupe de supports hydrophiles est représenté par les oxydes minéraux poreux dont la surface est revêtue par un polymère hydrophile naturel ou synthétique. L'oxyde minéral poreux peut être de la silice, de l'alumine, de la magnésie, un oxyde de titane, ou leurs dérivés naturels ou synthétiques tels que verres, silicates, zeolithes, kaolin, etc... Le support minéral possède une granulométrie de 4 µm à 5 mm, de préférence 50 µm à 1 mm, un diamètre poreux de 250 à 3000 Å, de préférence 600 à 1 500 Å, une surface spécifique de 5 à 150 $m^2$/g, de préférence 20 à 50 $m^2$/g.

Le polymère qui revêt la surface du support peut être un polymère naturel polysaccharidique tel que décrit ci-avant, il peut être un polymère hydrophile insoluble dsns l'eau, en lui-même connu, obtenu par polymérisation de monomères vinyliques en présence d'un agent de réticulation. Des exemples représentatifs de ces polymères hydrophiles comprennent les polymères d'acrylates et de méthacrylates d'hydroxyalkyle ou d'hydroxy(-alcoxy inférieur) alkyle tels que les homopolymères et copolymères d'acrylate ou de méthacrylate de 2-hydroxyéthyle, d'hydroxypropyle, d'hydroxyéthoxyéthyle; les polymères partiellement hydrolysés d'un ester vinylique d'acide carboxylique par exemple les homo- et copolymères partiellement hydrolysés d'acétate de vinyle; les polymères d'acrylamide; les polymères de composés vinyliques hétérocycliques tels que les homo - et copolymères de N-vinyllactames etc... Les oxydes minéraux revêtus de polymère polysaccharidique ont été décrits dans le brevet FR-A-2 319 399.

Les supports partiellement hydrophobes peuvent être constitués également de matrices de polymères naturels ou synthétiques ou bien d'oxydes minéraux tels que définis ci-avant revêtus d'un film de polymère partiellement hydrophobe. Les polymères polysaccharidiques peuvent être rendus partiellement hydrophobes par immobilisation de groupements tels que des radicaux hydrocarbonés alkyles ou alcényles, linéaires ou ramifiés, à un nombre de carbone égal ou supérieur à 3; des radicaux aryles ou alkylaryles tels que phényle, tolyle, xylyle; des radicaux cycloalkyle ou cycloalcényles tels que cyclohexyle. Les polymères synthétiques pouvent être obtenus par polymérisation en suspension de monomères vinylaromatiques, tels que styrène et dérivés, mis en oeuvre seuls ou en mélange entre eux et/ou avec au moins un monomère copolymérisable, tel que, par exemple, acrylates et méthacrylates d'alkyle ($C_1$-$C_5$), acrylonitrile, butadiène, et réticulés au moyen de monomères polyfonctionnels, tels que les diacrylates ou diméthacrylates de mono- ou poly-alkylèneglycols le divinylbenzène, les vinyltrialcoxysilanes, les vinyltrihalogénosilanes, le bis-méthylène acrylamide, en présence d'un initiateur ou de rayons ultraviolets.

Le support hydrophobe que l'on utilise préférentiellement selon le procédé de l'invention est constitué d'un oxyde minéral (tel que défini précédemment pour le support hydrophile) revêtu d'un film polymère au moins partiellement hydrophobe. Le polymère de revêtement peut être un polymère polysaccharidique sur lequel ont été immobilisés des groupements hydrophobes, par exemple des amines aliphatiques à longue chaîne (ce type de support peut être préparé selon le procédé décrit dans les brevets FR-A-2 403 098 ou US-A-4 308 254) ou bien un polymère synthétique obtenu à partir de monomères tels que les composés époxydiques, qui réticulent avec les polyamines comme catalyseurs; le formaldéhyde, qui réticule par polycondensation avec les polyamines; les monomères vinyliques: vinylpyridine, styrène et dérivés, qui réticulent avec des monomères polyfonctionnels comme les diacrylates ou diméthacrylates de mono- ou poly-alkylèneglycols, le divinylbenzène, les vinyltrialcoxysilanes, les vinyltrihalogénosilanes, le bis-méthylène acrylamide, en présence d'un initiateur ou de rayons ultraviolets.

Les groupes échangeurs d'ions peuvent être fixés sur le polymère hydrophile ou hydrophobe par modification chimique ou être introduits dans la chaîne polymérique par polymérisation ou copolymérisation à l'aide de monomères vinyliques portant des groupes fonctionnels ionisables de type acide ou de type basique, ou susceptibles d'être modifiés ultérieurement par réaction chimique pour introduire des radicaux échangeurs d'ions. Les monomères copolymérisables auxquels on peut faire appel sont des composés de formule:

$$H_2C=C(R_1)-R_2$$

dans laquelle $R_1$ est H ou $CH_3$ et $R_2$ représente:

- un radical aryl tel que phényl ou naphtényl
- un groupe fonctionnel réactif Y tel que $-C\equiv N$, $-CONH_2$, $-CH_2OH$, $-COOR_3$ où $R_3$ est H ou alkyl en $C_1$-$C_6$ ou

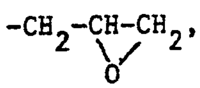, -OH,

$-NH_2$, -halogène, $-SO_3H$, $-PO(OH)_2$.

- ou un groupe fonctionnel Y ayant la signification précédente relié à l'atome de carbone insaturé par l'intermédiaire d'un radical divalent alkylène linéaire ou ramifié en $C_1$-$C_{30}$, phénylène, cycloalkylène, $-(CH_2)_n-O-(CH_2)_m-$, $-(CH_2)_n-CO-(CH_2)_m-$, $-(CH_2)_n-COO-(CH_2)_m-$, $-(CH_2)_n-SO_2-(CH_2)_m-$, $-(CH_2)_n-NR_4-(CH_2)_m-$, $-(CH_2)_n-CO-NR_4-(CH_2)_m$

où $R_4$ est H ou alkyle en $C_1$-$C_3$ et n, m est un nombre entier allant de 0 à 12.

Comme monomère copolymériaable, on peut citer l'acide (meth)acrylique, l'acide 4-pentenoïque, l'acide vinylbenzène sulfonique, l'acide vinylbenzène carboxylique, l'acrylamide et le methacrylamide, l'acrylonitrile et le méthacrylonitrile, l'alcool allylique, l'amine allylique, le 1-2 diméthyl 4-penténylamine, l'acrylate et le méthacrylate de 2-3 époxypropyle, le n.méthylolacrylamide, l'acide 6-acrylamidohexanoïque, le styrène.

Le choix du monomère vinylique réactif pour l'obtention du copolymère fonctionnel est seulement limité par l'utilisation qui sers faite du groupement fonctionnel. On peut par exemple choisir un monomère portant un groupe cationique ou un groupe anionique et utiliser directement le support comme résine échangeuse d'ions.

Si le monomère ne comporte pas lui-même de groupe échangeur d'ions, il est possible de le modifier ultérieurement par réaction chimique selon toute technique connue dans la mesure où cette réaction est compatible avec la stabilité du copolymère.

Il est également possible de modifier ultérieurement le groupe réactif Y présent dans le copolymère selon toute technique classique pour l'obtention d'un nouveau groupement fonctionnel Y'. Cette modification peut consister par exemple à transformer une amine primaire en amine tertiaire ou en sel d'ammonium quaternaire pour réaliser un support échangeur d'anions, ou à transformer un échangeur cationique en échangeur anionique par réaction sur un groupe -COOH d'une diamine que l'on transforme éventuellement en ammonium quaternaire par alkylation.

Le revêtement du support minéral par le polymère naturel ou synthétique est obtenu par toute méthode connue telle que pulvérisation ou imprégnation. Lorsque le polymère de revêtement est un polymère synthétique, le revêtement est préférentiellement obtenu par imprégnation du support avec une solution du ou des monomères énumérés ci-dessus et éventuellement de l'initiateur dans un solvant, qui est ensuite évaporé et les monomères réticulés selon les procédés connus. Comme solvant, on met en oeuvre tous produits solvants des monomères et de l'initiateur, dont le point d'ébullition est, de préference, le plus bas possible pour favoriser son évaporation ultérieure. Ce sont par exemple, le chlorure de méthylène, l'éther éthylique, le benzène, l'acétone, l'acétate d'éthyle, les chlorofluorométhanes (Flugènes).

De manière générale, la quantité de monomère(s) ou de polymère à mettre en oeuvre est choisie de manière à obtenir à la surface du support un film de polymère compris entre 1 et 15 $mg/m^2$ et de préférence entre 1 et 6 $mg/m^2$.

La quantité de groupements fonctionnels répartis à la surface du support est favorablement comprise entre 0,01 et 2 m.eq/g de support. Lorsque les supports sont utilisés exclusivement comme échange d'ions, cette quantité est préférablement comprise entre 0,05 et 2 meq/g de support.

Les groupes d'échanges d'anions peuvent être constitués par des groupes aminoaromatiques ou aminoaliphatiques tels que de préférence le groupe diéthylaminoéthyle ou des groupes aminoalcoyles quaternaires de formule $-N^{(+)}-(R)_3\ X^{(-)}$, dans laquelle R identique ou différent représente un groupe alkyle ou hydroxyalkyle et X représente un anion minéral ou organique tel que, par exemple, chlorure, sulfate, nitrate, phosphate, citrate, borate, acétate, formiate...

Les groupes d'échanges de cations peuvent être des groupes sulfonates, sulfate, phosphono, carboxyle ou hydroxyde phénolique, de préférence des groupes carboxyméthyle ou sulfoalcoyle.

Parmi les supports hydrophiles échangeurs d'ions disponibles commercialement, on peut citer les composés connus sous les dénominations SEPHADEX et SEPHAROSE vendus par la firme Pharmacia Fine Chemicals AB, notamment DEAE Sephadex (diéthylaminoéthyle dextran) QAE Sephadex (diéthylaminoéthyle dextran quaternisé) DEAE Sepharose (diéthylaminoéthyleagarose), CM Sephadex (carboxyméthyldextran) SP Sephadex (sulfopropyledextran), CM Sepharose (carboxymethylagarose) ainsi que les composés connus sous les dénominations DEAE Trisacryl M et CM Trisacryl M vendus par la Société Chimique Pointet-Girard.

Parmi les supports partiellement hydrophobes, on peut citer les produits connus sous les dénominations

PHENYL-SEPHAROSE CL-4B, OCTYL-SEPHAROSE CL-4B (Pharmacia Fine Chemicals AB) qui sont à base d'agarose réticulé; SPHEROSIL Q MA (Rhône-Poulenc Recherches) qui est une silice revêtue d'un polymère à base de vinyl toluène porteur de groupes ammonium quaternaire.

Le plasma que l'on met en oeuvre selon l'invention peut être d'origine animale, plasma bovin notamment, ou humaine. Il peut être obtenu par décantation ou centrifugation du sang ou par ultrafiltration sur des modules de plasmaphérèse. On peut mettre en oeuvre un plasma total frais ou décongelé, ou un surnageant de cryoprécipité ou, à fortiori des fractions issues d'un autre procédé de séparation.

Le plasma que l'on fractionne au moyen des échangeurs d'ions est préalablement traité pour le débarrasser au moins partiellement des sels et, avantageusement, des euglobulines qui constituent un complexe protéique instable. Ces traitements préliminaires ne nécessitent pas l'emploi d'adjuvants de clarification ou de précipitation tels que les polyéthylèneglycols ou d'agents d'adsorption comme les silices de combustion pulvérulentes. Mais bien entendu, le procédé de l'invention est applicable aux solutions plasmatiques ayant subi de tels traitements.

Le dessalage et l'ajustement des conditions de pH et force ionique peut être obtenu notamment par diafiltration ou chromatographie de perméation. Selon un aspect particulier de l'invention, le dessalage est réalisé par chromatographie de perméation sur un support minéral poreux dont les pores sont revêtus d'un film de polymère hydrophile tel que, par exemple, un polyvinyllactame réticulé. Le support minéral mis en oeuvre pour cette opération possède avantageusement un diamètre poreux de 40 à 300 Å (5 à 30 nm), une surface spécifique de 100 à 800 $m^2/g$ et une granulométrie de 4 µm à 5 mm.

Le revêtement peut être obtenu par simple adsorption du polymère sur le support, mais dans un mode de mise en oeuvre préféré, on effectue le revêtement par polymérisation in situ avec une solution de N-vinyllactame, par exemple la N-vinylpyrrolidone, en présence d'un agent de réticulation polyfonctionnel choisi parmi les composés décrits précédemment et de la manière décrite pour la préparation de la résine échangeuse de cations. Parmi les réticulants, on préfère mettre en oeuvre un dérivé silanique qui permet de former une liaison stable entre le support minéral et le polyvinyllactame. La quantité de monomères est choisie de façon à obtenir à la surface du support un film de polymère compris entre 1 et 15 $mg/m^2$, de préférence entre 1 et 6 $mg/m^2$, la quantité de réticulant pouvant varier entre 1 et 50 % en poids par rapport au poids total des monomères.

A faible force ionique le plasma peut être débarrassé des Euglobulines qu'il contient à un pH voisin de 5, puis centrifugation et séparation de la solution de plasma clarifié.

Selon le procédé de l'invention, la solution de plasma est mise en contact avec au moins un support partiellement hydrophobe et au moins un support hydrophile. Le support partiellement hydrophobe ne contient pas nécessairement de groupes échangeurs d'ions mais il est nécessaire de mettre en oeuvre au moins un support échangeur d'anions et au moins un support échangeur de cations. Avantageusement l'échangeur d'anions précède l'échangeur de cations.

Dans un mode de réalisation préférentiel, pour l'obtention d'un meilleur rendement et d'une albumine de plus grande pureté, le support partiellement hydrophobe est échangeur d'anions et le support hydrophile est échangeur de cations.

Dans le mode de réalisation le plus préférentiel, on conduit la séparation en associant deux échangeurs d'anions dont l'un peut être hydrophile et l'autre est partiellement hydrophobe, et un échangeur de cations hydrophile. L'ordre de passage sur les deux derniers supports est indifférent.

Le fractionnement du plasma ajusté en force ionique, et de préférence clarifié, se fait successivement avec le ou les échangeurs d'anions et avec l'échangeur de cations en réglant le pH et la force ionique de manière à fixer sélectivement soit l'albumine, soit les autres protéines ou impuretés.

Par exemple, la solution de plasma peut être mise en contact avec le support échangeur d'anions partiellement hydrophobe, préalablement équilibré pour fixer principalement l'albumine et les α globulines à un pH égal ou supérieur à 5. Par élution au moyen d'une solution tampon de pH compris entre 4,4 et 4,8 et de force ionique convenablement choisie, on obtient une solution riche en albumine, contenant des α et β globulines résiduelles. A titre indicatif, on peut utiliser un pH de 4,7 et tampon acétate 0,025 M. La solution éluée, ajustée à un pH supérieur ou égal à 5,0 est ensuite mise en contact avec l'échangeur de cations préalablement équilibré avec un tampon de pH identique sur lequel est retenue la quasi-totalité des protéines autres que l'albumine.

Dans un autre exemple de mise en oeuvre, la solution de plasma est mise en contact avec successivement un échangeur d'anions hydrophile, un support partiellement hydrophobe, de préférence échangeur d'anions, puis un échangeur de cations hydrophile. Le pH et la force ionique de la solution sont réglés de la manière ci-dessus indiquée pour retenir l'albumine et certaines α et β globulines sur le premier échangeur d'anions hydrophile. Après élution, la solution contenant l'albumine est mise en contact avec le support partiellement hydrophobe sur lequel diverses impuretés dont les lipoprotéines peuvent être fixées à un pH de 4,5 à 6,0 et de préférence 4,7 à 5,2 avec un tampon acétate 0,025 à 0,06 M. La solution éluée contenant l'albumine est finalement purifiée sur l'échangeur de cations dans les conditions ci-dessus indiquées.

Les immunoglobulines qui traversent le support échangeur d'anions peuvent être purifiées par mise en contact avec un autre support échangeur d'ions en faisant varier le pH et la force ionique de la solution.

Dans le cas préféré où l'on met en oeuvre des supports à base d'oxydes minéraux, les quantités d'échangeur d'anions peuvent être comprises entre 250 et 2000 ml pour 1 litre de plasma et les quantités d'échangeur de cations peuvent être comprises entre 200 et 1000 ml. Le temps de contact de la solution avec chacun des

échangeurs est supérieur à environ 15 minutes.

Le procédé de l'invention permet de valoriser la totalité des protéines contenues dans le plasma initial et d'obtenir une albumine en solution dont la pureté, déterminée par électrophorèse à une concentration de 50 g/litre est supérieure ou égale à 99 % et peut atteindre 100 %. L'analyse par filtration sur gel montre l'absence de polymères de l'albumine. La pureté de la solution d'albumine peut être évaluée par les techniques d'immunoprécipitation. En particulier, selon la technique d'immunodiffusion double en gélose, on peut montrer l'absence de lipoprotéines $\alpha 1$ et $\beta$; des traces d'$\alpha 1$ antitrypsine et d'haptoglobine éventuellement détectables sont sans inconvénient pour la qualité de l'albumine. Cette solution d'albumine peut être concentrée par tous procédés connus, par exemple par ultrafiltration, puis ajustée et stabilisée pour être soumise au chauffage légal de 10 heures à 60°C, en vue de la rendre injectable. Les protéines autres que l'albumine, qui sont retenues par les échangeurs peuvent être éluées au moyen d'une solution de régénération. Les protéines en mélange contenues dans les solutions effluente et de régénération peuvent être ultérieurement séparées les unes des autres par des techniques chromatographiques.

Le traitement du plasma peut être effectué en cuve en discontinu, en semi-continu ou en continu avec des séries de colonnes, cette dernière possibilité étant particulièrement adaptée aux réalisations industrielles. Le procédé en continu en colonnes permet d'obtenir une meilleure productivité, un meilleur rendement et une plus grande pureté des produits dans des conditions de stérilité plus favorables.

On donne ci-après, à titre indicatif et non limitatif, des exemples de réalisation de l'invention.

**Exemple 1**

On prépare:

A - <u>un support pour chromatoghie d'exclusion (GPC)</u>

200 g de silice ayant une granulométrie de 40 à 100 μm, une surface spécifique de 400 $m^2/g$, un diamètre poreux moyen de 80 Å et un volume poreux de 1 ml/g sont séchés à 150°C sous pression réduite pendant 5 heures.

La silice sèche obtenue est introduite dans une solution homogène contenant 500 ml de flugène 11 ($CCl_3F$), 160 ml de N-vinyl pyrrolidone, 30 ml de vinyltriéthoxysilane et 1 g de péroxyde de dilauroyle. Le flugène 11 est évaporé à température ambiante puis la silice imprégnée est chauffée à 80°C pendant 17 h pour obtenir la réticulation.

La silice est alors mise en suspension dans 800 ml d'eau permutée et portée à l'ébullition pendant 5 h. Après filtration, la silice est lavée à l'eau permutée, à l'acétone puis séchée sous vide à 40°C. L'analyse donne un taux de carbone de 12 % en poids par rapport à la silice revêtue.

B - <u>un échangeur d'anions sur support</u>

constitué d'une silice ayant une granulométrie de 100 à 300 μm, une surface spécifique de 25 $m^2/g$, un diamètre poreux moyen de 1.250 Å, un volume poreux de 1 ml/g, revêtue de 3,6 $mg/m^2$ d'un copolymère obtenu par réticulation du vinyltoluène portant des groupes fonctionnels $-N^{(+)}-(CH_3)_3\ Cl^{(-)}$ et présentant les caractéristiques suivantes:

| | |
|---|---|
| Taux de carbone | 6,1 % |
| Taux d'azote | 0,42 % |
| Capacité ionique | 0,30 m.eq/g |

C - <u>un échangeur de cations sur support</u>

100 g de silice ayant une granulométrie de 100 à 300 μm, une surface spécifique de 25 $m^2/g$, un diamètre poreux moyen de 1.250 Å et un volume poreux de 1 ml/g sont séchés à 150°C sous pression réduite pendant 5 h.

La silice sèche obtenue est introduite dans une solution de 250 ml de flugène 11 dans laquelle ont été homogénéisés 50 ml de N-vinylpyrrolidone, 2 ml d'acide acrylique, 15 ml de vinyltriéthoxysilane et 0,50 g de péroxyde de dilauroyle. Après avoir évaporé le solvant à température ambiante, la silice imprégnée est chauffée à 80°C pendant 16 h pour obtenir la réticulation.

La silice est alors mise en suspension dans 400 ml d'eau permutée et portée à l'ébullition pendant 5 h. Après filtration, la silice est lavée à l'eau permutée, à l'acétone puis séchée sous vide à 40°C.

La silice greffée échangeuse de cations faible ainsi obtenue présente les caractéristiques suivantes:

% C 6,05
% H 1,09
% N 0,3
Capacité ionique: 0,20 m.eq/g
Quantité de polymère fixé: 3,2 $mg/m^2$

**Prétraitement du plasma**

Dans une colonne de 2,6 cm de diamètre sont placés et maintenus comprimés 100 g du support A.

Sur cette colonne sont injectés successivement à un débit de 30 ml/h, 200 ml de tampon acétate de sodium 0,025 M, 60 ml de plasma frais provenant d'un module de plasmapherèse et à nouveau 100 ml de tampon acétate 0,025 M. La totalité des protéines est collectée dans 65 ml de solution dont la résistivité est voisine de celle du tampon utilisé.

Le pH de la solution protéique est ajusté à 5,1 par addition lente d'une solution d'acide acétique à 6 %. Les euglobulines précipitées sont séparées par centrifugation.

La solution protéique ainsi obtenue présente les caractéristiques suivantes:

Résistivité: 520 Ω $cm^2/cm$

Protéines totales: 43,8 g/litre avec la composition suivante:

| | |
|---|---|
| Albumine | 66,8 % |
| α globulines | 9,2 % |
| β globulines | 6,6 % |
| γ globulines | 17,4 % |

**Fractionnement de la solution plasmatique**

Dans une colonne I de 1,6 cm de diamètre sont placés et maintenus comprimés 25 g de l'échangeur d'anions B. La colonne est équilibrée avec un tampon acétate de sodium 0,025 M à pH: 5,1.

Dans une colonne II de 1 cm de diamètre, sont placés et maintenus comprimés 10 g de l' échangeur d'anions B. Cette colonne est équilibrée avec un tampon acétate de sodium 0,025 M à pH: 4,7.

Dans une colonne III de 1 cm de diamètre sont placés et maintenus comprimés 5 g de l'échangeur de cations C. Cette colonne est équilibrée avec un tampon acétate de sodium 0,1 M à pH: 5,5.

Dans la colonne I sont injectés successivement 58 ml de la solution plasmatique prétraitée à un débit constant de 70 ml/h, 100 ml de la solution tampon acétate de sodium 0,025 M à pH 5,1 puis une solution tampon acétate 0,025 M à pH: 4,7.

On récupère 185 ml d'une solution appelée éluat de la colonne I dont la teneur en protéines totales est de 8,8 g/l ét qui présente la composition suivante:

| | |
|---|---|
| Albumine | 93 % |
| α globulines | 1,6 % |
| β globulines | 3 % |
| γ globulines | 2,4 % |

Le rendement d'extraction en albumine de cette colonne est de 89,2 %.

130 ml de l'éluat de la colonne I sont injectés à un débit constant de 40 ml/h dans la colonne II. 110 ml de tampon acétate 0,025 M à pH: 4,7 sont ensuite injectés dans la colonne II. Les solutions effluente et de lavage sont mélangées pour donner une solution dont la teneur en protéines totales est de 4,2 g/l avec la composition suivante:

| | |
|---|---|
| Albumine | 96,5 % |
| α globuline | 0,8 % |
| β globuline | 2,7 % |

Le rendement d'extraction en albumine sur la colonne II est de 91,4 %.

La solution collectée dans la colonne II est ajustée à pH: 5,5 et à la résistivité de 180 Ω $cm^2/cm$ par addition de chlorure de sodium. 94 ml de cette solution sont injectés dans la colonne III à un débit de 40 ml/h. La colonne est lavée avec 26 ml de tampon acétate 0,1 M à pH: 5,5. Les solutions effluente et de lavage sont mélangées pour donner une solution dont la teneur en protéines totales est de 3,0 g/l et dont la composition est la suivante:

| | |
|---|---|
| Albumine | 99,5 % |
| α globuline | 0,5 % |

Le rendement d'extraction en albumine de cette colonne III est de 94 %.

Le rendement d'extraction en albumine obtenu par l'ensemble du fractionnement est de 76,6 %.

Les colonnes I et II peuvent être régénérées avec un tampon acétate de sodium 1 M à pH: 4,0 tandis que la colonne III peut être régénérée avec une solution HCl - 0,5 N.

Le filtrat (ou solution effluente) de la colonne I contient 75 % de γ globulines et 25 % de β globulines avec des traces de α globulines. Ce filtrat est injecté sur une colonne IV, identique à la colonne I, après ajustement à un pH de 5,8 et une force ionique 0,050 M. On recueille une solution dont la pureté en γ globulines est voisine de 100 %. Le rendement global d'extraction en γ globulines est de 80 %.

**Exemple 2**

100 ml de plasma stocké à -40°C provenant d'un module de plasmaphérèse est décongelé lentement à +4°C et centrifugé pour éliminer le précipité contenant le facteur VIII. Le plasma obtenu "surnageant de cryoprécipité" est dialysé par diafiltration contre un tampon acétate 0,005 M à pH 7,4. Après acidification de la solution à pH 5,1 par de l'acide acétique à 6 % puis centrifugation pour éliminer les euglobulines, la solution protéique obtenue présente les caractéristiques suivantes:

Résistivité: 1.066 Ω $cm^2/cm$

Protéines totales: 39,4 g/litre avec la composition:

| | |
|---|---|
| Albumine | 67,3 % |
| α globulines | 9,1 % |
| β globulines | 7,2 % |
| γ globulines | 16,3 % |

**Fractionnement du plasma**

58 ml de la solution protéique ci-avant sont injectés à un débit de 70 ml/h sur la colonne I décrite à l'exemple 1, contenant l'échangeur d'anions B et préalablement équilibrée avec un tampon acétate de sodium 0,025 M à pH 5,1.

La colonne est lavée avec 100 ml de la même solution tampon puis éluée avec une solution tampon acétate 0,025 M à pH 4,5.

L'éluat ainsi collecté (125 ml) dont la teneur en protéines totales est de 10,1 g/l présente la composition suivante:

| | |
|---|---|
| Albumine | 95,0 % |
| α globulines | 1,8 % |
| β globulines | 3,2 % |
| γ globulines | 0 % |

Le rendement d'extraction en albumine de cette colonne est de 78 %.

50 ml de l'éluat sont injectés à un débit constant de 40 ml/h sur une deuxième colonne identique à la colonne III de l'exemple 1 contenant l'échangeur de cations C et préalablement équilibrée avec un tampon acétate 0,1 M à pH 5,5. La colonne est lavée avec 15 ml du même tampon. Les solutions effluente et de lavage sont mélangées pour donner une solution dont la teneur en protéines totales est de 7,27 g/l avec la composition suivante:

| | |
|---|---|
| Albumine | 99,0 % |
| α globulines | 1,0 % |

Le rendement en albumine de cette colonne est de 97,5 %, le rendement global d'extraction en albumine obtenu par le procédé de fractionnement est de 76 %.

**Exemple 3**

100 ml de plasma bovin obtenu par centrifugation sont dialysés par diafiltration contre un tampon acétate 0,005 M à pH: 7,4.

La solution protéique obtenue après élimination des euglobulines, comme décrit dans les exemples précédents a une résistivité de 1.164 Ω $cm^2/cm$ et une teneur en protéines totales de 39,1 g/l avec la composition suivante:

| | |
|---|---|
| Albumine | 66,1 % |
| α globulines | 8,0 % |
| β globulines | 10,0 % |
| γ globulines | 15,9 % |

58 ml de la solution protéique sont injectés à un débit de 70 ml/h sur la colonne I décrite à l'exemple I contenant l'échangeur d'anions B et équilibrée avec un tampon acétate 0,025 M à ph 5,1. La colonne est traitée comme dans l'exemple précédent. L'éluat collecté (167 ml) renferme 7,5 g/l de protéines dont la composition est la suivante:

| | |
|---|---|
| Albumine | 95,3 % |
| α globulines | 1,6 % |
| β globulines | 3,1 % |
| γ globulines | 0 |

67 ml de l'éluat sont ensuite injectés à un débit de 40 ml/h sur une seconde colonne identique la colonne III décrite à l'exemple 1 contenant l'échangeur de cations et équilibrée avec un tampon acétate 0,1 M à pH 5,5. La colonne est lavée avec 23 ml du même tampon.

Les solutions éluente et de lavage sont mélangées. On obtient une solution dont la teneur en protéines totales est de 5,1 g/l avec la composition suivante:

| | |
|---|---|
| Albumine | 99,1 % |
| α globulines | 0,9 % |

Le rendement en albumine obtenu par le procédé de fractionnement est de 75,6 %.

**Exemple 4**

**Préparatiton de la solutiton plasmatique**

250 ml de plasma sont décongelés à 37°C, ajustés à pH=5,2 avec HCl concentré et dialysés contre 60 l de tampon phosphate 0,01 M à pH=5,2.

Après centrifugation, on obtient 293 ml d'une solution plasmatique ayant une concentration en protéines totales de 35,5 g/l avec la composition suivante:

| | |
|---|---|
| Albumine | 56,5 % |
| α globulines | 12,9 % |
| β globulines | 9,5 % |
| γ globulines | 21,1 % |

**Fractionnement**

On installe en série:

- une colonne I (Ø=1,6 cm) contenant 15 g du support d'échangeur d'anions partiellement hydrophobe B décrit à l'exemple 1
- une colonne II (Ø=1 cm) contenant 5 g du même échangeur d'anions B
- une colonne III (Ø=1 cm) contenant 5 g d'un support d'échangeur de cations D constitué d'une silice ayant une granulométrie de 100 à 300 µm, une surface spécifique de 32 $m^2/g$, un diamètre poreux moyen de 1150 Å et un volume poreux de 1,2 ml/g, revêtu de 5 $mg/m^2$ d'un copolymère N-vinyl-pyrrolidone-acide acrylique. Ce support est préparé de la manière décrite à l'exemple 1 pour l'échangeur de cations C en modifiant le rapport des monomères. Il présente les caractéristiques suivantes:

| | |
|---|---|
| - taux de carbone | 8,46 % |
| - taux d'azote | 1,29 % |
| - capacité ionique | 0,19 meq/g |

35 ml de la solution plasmatique préparée ci-avant sont injectés à un débit de 60 ml/heure sur la colonne I préalablement équilibrée avec un tampon acétate 0,025 M à pH=5,2. Après avoir lavé la colonne avec 65 ml de la solution tampon d'équilibrage, on injecte au même débit (60 ml/h) un tampon acétate 0,025 M et pH 4,7. La solution éluée 160 ml (concentration en protéines 3,72 g/l) titre 92,5 % en albumine, 3 % en α globulines et 4,5 % en β + γ globulines. Une fraction aliquote (138 ml) de la solution éluée est injectée à un débit de 40 ml/h sur la colonne II préalablement équilibrée avec le tampon acétate 0,025 M pH 4,7. La colonne est lavée avec 22 ml de la même solution tampon. Le mélange des solutions effluente et de lavage, dont la concentration en protéines est de 3,06 g/l titre 93,6 % en albumine, 2,5 % en α globulines et 3,5 % en β globulines.

Une fraction aliquote (140 ml) du mélange des solutions précédentes est ajustée à pH 5,5 par addition de NaOH et à une résistivité de 270 Ω $cm^2/cm$ par addition de NaCl 1M puis injectée à un débit de 20 ml/h sur la colonne III préalablement équilibrée avec un tampon acétate 0,05 M, pH 5,5. La colonne est lavée avec 25 ml de la même solution tampon.

L'électrophorèse effectuée sur le mélange reconcentré des solutions effluente et de lavage montre l'absence d'impuretés contaminantes de l'albumine (albumine 100 %).

Le rendement global est de 71 % par rapport à l'albumine contenue dans la solution plasmatique de départ.

Les colonnes I, II et III sont régénérées par lavage avec une solution HCl 0,1 N, eau distillée et solution tampon respective.

**Exemple 5**

On prépare:

A - un échangeur d'anions hydrophile sur support minéral poreux dénommé "Echangeur d'anions E".

100 g de silice poreuse (SPHEROSIL XOB 015) de granulométrie 100-200 μ, de surface spécifique 30 $m^2/g$, de diamètre poreux moyen 1250 Å (125 nm), de volume poreux 1 ml/g sont additionnés de 200 ml d'une solution aqueuse de DEAE Dextran à 7,5 %, ajustée à pH 11,5 par addition d'hydroxyde de sodium.

La pâte est séchée dans une étuve à plateaux à 80°C pendant 15 heures. La poudre obtenue est additionnée de 300 ml d'une solution à 0,15 % de 1-4 butane diol-diglycidylether dans l'éther éthylique. L'éther est évaporé sous courant d'azote à température ambiante. La réaction de réticulation est ensuite réalisée par chauffage à 80°C pendant 15 heures.

Avant utilisation, ce support est lavé par les solutions suivantes: 10 volumes de NaOH 0,1 N, 10 volumes d'HCl 0,1 N et 10 volumes d'alcool éthylique puis séché pour être stocké avant emploi.

On obtient 115 g de poudre contenant 13 % de DEAE Dextran et capable de fixer 0,2 meq d'ions Cl par gramme.

B - un échangeur d'anions partiellement hydrophobe, sur support minéral poreux, dénommé "Echangeur d'anions F".

Le support servant à sa préparation est préparé comme en A ci-dessus, mais ne contient que 5,7 % de DEAE Dextran en final.

100 g du support sont oxydés par 500 ml d'une solution aqueuse de métapériodate de sodium $NaIO_4$ 0,05 M contenant 10 g/l NaCl, pendant 2 h à température ambiante. Après lavages par une solution de NaCl 10 g/l, puis d'alcool éthylique, le produit est séché sous courant d'azote à 40°C.

100 g du support oxydé sont additionnés de 100 ml d'une solution d'hexadecylamine à 6 % dans l'alcool éthylique et laissés à température ambiante pendant 48 heures.

La réduction de la liaison imine ainsi fomée, en liaison amine s'effectue par addition d'hydroborure de sodium $NaBH_4$ pour atteindre une concentration de 0,2 M, en présence de 50 % d'eau. Après des lavages en alcool, puis en HCl 0,1 N répétés, le support lavé est séché pour être stocké avant emploi.

**Prétraitement du plasma**

200 ml de plasma humain prélavé sur citrate de sodium sont additionnés de 200 ml d'une solution aqueuse d'éthanol à 16 % et à 0°C. Le précipité formé est éliminé par centrifugation. Le surnageant est acidifié (par HCl N) à pH 5,25 à +4°C. Après 2 heures, le précipité formé est éliminé par centrifugation et le surnageant est diafiltré dans une cellule d'ultrafiltration pour être équilibré avec un tampon Phosphate 0,01 M pH 5,25. Le précipité d'euglobulines est à nouveau éliminé par centrifugation et le surnageant obtenu est filtré sur membrane de porosité 0,2 μ. Sa résistivité est de 1200 Ω $cm^2/cm$.

**Fractionnement de la solution plasmatique**

- Une colonne 1 de 2,5 cm de diamètre est remplie avec 50 g (105 ml) de l'échangeur d'anions E. La colonne est équilibrée avec un tampon phosphate de sodtum 0,01 M à pH 5,25.
- Une colonne II de 2,5 cm de diamètre est remplie avec 30 g (63 ml) de l'échangeur d'anions F. La colonne est équilibrée avec un tampon acétate de sodium 0,054 M à pH 5.
- Une colonne III de 2,5 cm de diamètre est remplie avec 60 g (126 ml) de l'échangeur de cations D décrit dans l'exemple 4. La colonne est équilibrée avec un tampon acétate de sodium 0,054 M à pH 5,5.

La solution clarifiée correspondant à 200 ml de plasma, obtenue selon le procédé de prétraitement décrit précédemment, est injectée dans la colonne I à un débit de 400 ml/h. La colonne est rincée avec 200 ml de tampon phosphate 0, 01 M pH 5,25, puis l'albumine fixée est éluée avec 500 ml de tampon acétate 0,025 M pH 4,7. L'éluat est collecté, son volume est de 400 ml.

L'éluat ainsi obtenu est ajusté à pH 5 par addition de NaOH N puis additionné de NaCl pour atteindre une résistivité de 270 Ω $cm^2/cm$. Cette solution est injectée sur la colonne II à un débit de 100 ml par heure. La majorité de l'albumine n'est pas fixée dans ces conditions et traverse la colonne avec le filtrat. La colonne est rincée par 120 ml d'acétate de sodium 0,054 M pH 5.

Le mélange contenant l'albumine est ajusté à pH 5,5 par addition de NaOH et injecté sur la colonne III à un débit de 200 ml/h. La colonne est rincée par 200 ml de tampon acétate de sodium 0,054 M pH 5,5. Les solutions effluentes sont mélangées et concentrées par ultrafiltration sur une membrane AMICON PM 10 pour atteindre une concentration de 50 à 200 g/l. La pureté de la solution d'albumine à 50 g/l est estimée par électrophorèse et immunoélectrophorèse sur acétate de cellulose dans les conditions de la Pharmacopée. Elle est de 100 %. L'analyse par immunodiffusion double en gélose vis-à-vis d'antisérums spécifiques des diverses protéines plasmatiques permet de vérifier l'absence d'impuretés protéiques et notamment d'α et β lipoprotéines. Lorsque la contentration finale de l'albumine purifiée est de 130 g/l, des traces d'α1 antitrypsine et d'haptoglobine non visibles à l'électrophorèse peuvent être détectées par immunodiffusion double vis-à-vis des antisérums correspondants.

La présence de ces traces d'impuretés est normale et sans conséquence sur la stabilité de la solution d'albumine finale, ou sur sa bonne tolérance lors d'administration intraveineuse chez l'animal ou chez l'homme.

Les résultats concernant le rendement et la pureté de l'albumine sont résumés dans le tableau I.

L'ensemble des trois colonnes peut être lavé par une solution d'acétate 0,1 M pH 4, ou de NaCl 20 g/l qui permet de récupérer l'albumine retenue pour la recycler lors du cycle suivant ou dans un traitement indépendant.

Les colonnes sont ensuite régénérées par lavages successifs en HCl 0,1 N et Ethanol 60 %. Elles peuvent être éventuellement stérilisées par voie humide (Formol 2 %), périodiquement.

**Exemple 6**

Dans cet exemple, on utilise un support échangeur d'anions hydrophile, un support échangeur d'anions hydrophobe et un support échangeur de cations hydrophile.

On dispose en série:

- une colonne I (Ø=1,6 cm) contenant 14 g du support échangeur d'anions E décrit à l'exemple 5. La colonne est équilibrée avec un tampon phosphate 0,01 M à pH=5,2
- une colonne II identique à la colonne II de l'exemple 4 contenant l'échangeur d'anions B
- une colonne III identique à la colonne III de l'exemple 4 contenant l'échangeur de cations D.

On opère comme dans l'exemple 4 à partir de 61 ml de la même solution plasmatique et en utilisant les mêmes solutions tampon pour l'élution de la colonne I et le lavage des colonnes II et III.

Les débits des colonnes I, II et III sont respectivement de 60 ml/h, 20 ml/h et 20 ml/h.

L'analyse par électrophorèse de la solution finale, concentrée à 50 g/l, montre l'absence d'impuretés contaminantes de l'albumine. Les contrôles en immunodiffusion double de la solution contentrée à 130 g/l montrent l'absente de toute impureté protéique.

Les % d'albumine et rendements des colonnes sont mesurés dans le Tableau I.

**Exemple 7**

On opère comme dans l'exemple 6 en inversant l'ordre des colonnes II et III.

L'éluat sortant de la colonne I est ajusté à pH=5,5 avec NaOH et à une résistivité de 270 Ω $cm^2$/cm avet NaCl 1 M pour être injecté dans la colonne II.

La solution (effluente + lavage) venant de la colonne II est ajustée à pH=4,7 avet HCl 1 N pour être injectée dans la colonne III.

Les débits sont de 60 ml/h, 20 ml/h et 20 ml/h respectivement.

L'analyse par électrophorèse et immunodiffusion de la solution finale, concentrée à 130 g/l, montre l'absence d'impuretés contaminantes de l'albumine.

Les résultats, % albumine et rendement, sont résumés dans le Tableau I.

**Exemple 8**

On opère comme dans l'exemple 6 mais la colonne II est remplacée par une nouvelle colonne II contenant un support partiellement hydrophobe non échangeur d'ions. Ce support est constitué d'une silice ayant une granulométrie de 100 à 300 µm, une surface spécifique de 25 $m^2$/g, un diamètre poreux moyen de 1250 Å et un volume poreux de 1 ml/g revêtu de 3,3 mg/$m^2$ d'un polymère réticulé vinyltoluènevinyltriéthoxysilane et présentant un taux de tarbone de 7,3.

La colonne II est équilibrée avec 50 ml d'éthanol à 95 % puis 50 ml d'une solution tampon acétate 0,025 M à pH 4,7.

Les débits des colonnes I, II et III sont de 60 ml/h, 20 ml/h et 20 ml/h respectivement.

La solution effluente de la colonne III titre 99,3 % d'albumine et 0,7 % d'α globulines.

Les résultats figurent dans le Tableau I.

**Exemple 9**

On opère dans les mêmes conditions que dans l'exemple 5 en remplaçant la colonne III par une colonne de CM SEPHAROSE CL-6B (Pharmacia Fine Chemicals, à Uppsala-Suède) de même taille et dans les mêmes conditions d'emploi. Les résultats figurent au Tableau I.

**Exemple 10**

On opère dans les mêmes conditions que dans l'exemple 6 en remplaçant la colonne III par une colonne de CM Trisatryl-M (Réactifs IBF, Société POINTET-GIRARD à Villeneuve la Garenne, France) de même taille et dans les mêmes conditions d'emploi.
Les résultats figurent dans le Tableau I.

**Exemple 11**

On opère dans les mêmes conditions que l'exemple 9 en remplaçant la colonne I par une colonne de DEAE Sepharose CL 6B (Pharmacia Fine Chemicals à Uppsala en Suède) de même taille et dans les mêmes conditions d'emploi.
Les résultats figurent dans le Tableau I.

**Exemple 12**

On opère dans les mêmes conditions que l'exemple 10 en remplaçant la colonne I par une colonne de DEAE Trisacryl M (Réactifs IBF, Société POINTET-GIRARD à Villeneuve La Garenne, France) de même taille et dans les mêmes conditions d'emploi.
Les résultats figurent dans le Tableau I.

**Exemple 13**

- Une colonne I de 2,5 cm de diamètre est remplie avet 100 g (210 ml) de l'échangeur d'anions E décrit à l'exemple 5. La colonne est équilibrée avec un tampon acétate de sodium 0,025 M pH 5,25.
- Une colonne II de 2,5 cm de diamètre est remplie avec 63 ml de SEPHAROSE CL 4B greffé avec l'hexadecylamine et équilibrée en acétate de sodium 0,054 M pH 5. Le greffage de l'hexadecylamine s'effectue par la méthode au bromure de cyanogène selon la procédure suivante:
100 ml de SEPHAROSE CL 4B sont lavés en eau déminéralisée par 3 décantations successives et additionnés de 200 ml d'eau. Le pH étant maintenu à 11 par addition continue de soude (NaOH), 9 g de BrCN sont ajoutés. La réaction dure environ 15 minutes. Lorsque le pH n'évolue plus, le support est rincé en bicarbonate 0,1 M puis en alcool éthylique à 95 %.
20 g d'hexadetylamine dissous dans 200 ml d'alcool sont additionnés au support à 40° C, la réaction évolue pendant 15 heures.
En final, le support est lavé plusieurs fois successivement par des solutions d'HCl 0,1 N et d'alcool 95 %, puis équilibré comme indiqué.
- Une colonne III de 2,5 cm de diamètre est remplie avec 60 g (126 ml) de l'échangeur de cations D détrit dans l'exemple 4. La colonne est équilibrée avec un tampon acétate de sodium 0,054 M pH 5,5.

**Prétraitement du plasma humain**

200 ml de surnageant de cryoprécipité sont additionnés de 200 ml d'une solution aqueuse d'éthanol à 16 % et à 0°C. Le précipité formé est éliminé par centrifugation. Le surnageant est acidifié (par HCl N) à pH 5,25 à +4°C. Après 2 heures, le précipité formé est éliminé par centrifugation et le surnageant est diafiltré dans une cellule d'ultrafiltration pour être équilibé avec un tampon acétate 0,025 M pH 5,25. Le précipité d'euglobulines est à nouveau éliminé par centrifugation et le surnageant obtenu est filtré sur membrane de porosité 0,2 μ. Sa résistivité est de 630 Ω cm²/cm. La solution est injectée dans la colonne I à un débit de 400 ml/h. La colonne est rincée avet 200 ml de tampon acétate 0,025 M pH 5,25, puis l'albumine fixée eat éluée avec 400 ml de tampon acétate 0,025 M pH 4,7. L'éluat est collecté, son volume est de 350 ml.
L'éluat ainsi obtenu est ajusté à pH 5 par addition de NaOH puis additionné de NaCl pour atteindre une résistivité de 270 Ω cm²/cm. La solution est injectée sur la colonne II à un débit de 100 ml/h.
Une quantité non négligeable d'albumine est fixée par la colonne, diminuant le rendement de 20 à 30 % par rapport aux supports utilisés en colonne n° II dans les exemples précédents. La solution qui traverse la colonne contient de l'albumine et quelques α et β globulines. La colonne est rincée par 120 ml d'acétate de sodium 0,054 M pH 5. Le mélange contenant l'albumine est ajusté à pH 5,5 par addition de NaOH et injecté sur la colonne III à un débit de 200 ml/h. La colonne est rincée par 200 ml de tampon acétate de sodium 0,054 M pH 5,5. Les solutions effluentes sont mélangées et concentrées par ultrafiltration sur une membrane AMICON PM 10 pour atteindre une concentration de 50 g/l.
La pureté de la solution, estimée par électrophorèse en acétate de cellulose dans les conditions de la

Pharmacopée est de 99,1 % et peut donc être considérée comme satisfaisante d'après les normes en vigueur. L'analyse en immunodiffusion double en gélose vis-à-vis d'antisérums spécifiques des diverses protéines plasmatiques permet de détecter la présence des impuretés suivantes: transferine, α1 antitrypsine, α2 macroglobuline, haptoglobine.

Les trois colonnes peuvent être régénérées et stérilisées entre chaque cycle comme dans les exemples précédents.

Le rendement global de ce procédé de purification est seulement de 55 %.

Les résultats figurent dans le Tableau I.

**Exemple 14 (Comparatif)**

On opère dans les mêmes conditions que dans l'exemple 13 mais en ramplaçant la colonne II de SEPHAROSE hydrophobe par une colonne d'échangeur d'anions E, hydrophile, dans les mêmes conditions d'emploi. Les résultats obtenus (voir Tableau I) sont différents de ceux des exemples précédents. La pureté de l'albumine finale est inférieure à 99 %. On détecte notamment la présence systématique d'$\alpha^1$ lipoprotéine dont la présence nuit à la stabilité de la solution d'albumine finale, notamment lors du chauffage à 60° C.

Cet exemple démontre la nécessité d'inclure une étape de filtration sur un support partiellement hydrophobe, pour obtenir une albumine de très haute pureté.

**Tableau I**

| | Albumine % (électrophorèse) | | | Rendements en albumine % | | | |
|---|---|---|---|---|---|---|---|
| Ex. | Eluat COL. I | Après COL. II | Après COL. III | COL. I | COL. II | COL. III | Total |
| 4 | 92,5 | 93,6 | 100 | 78,5 | 96,5 | 93,6 | 71 |
| 5 | 93,7 | 96,5 | 100 | 79 | 92 | 98 | 71 |
| 6 | 93,7 | 96,2 | 100 | 79 | 91,3 | 98 | 71 |
| 7 | 93,7 | 96,6 | 100 | | | | 78 |
| 8 | 93,7 | 94,9 | 99,3 | | | | 67 |
| 9 | 92,8 | 96,5 | 100 | | | | 75 |
| 10 | 94 | 96,8 | 100 | | | | 72 |
| 11 | 92,5 | 96,2 | 100 | | | | 69 |
| 12 | 93,0 | 96,5 | 100 | | | | 73 |
| 13 | 94,2 | 94,7 | 99,1 | | | | 55 |
| 14 | 94,2 | 94,3 | 97 | | | | 79 |

**Revendications**

1. Procédé de fractionnement du plasma au moyen d'échangeurs d'ions, anionique et cationique, caractérisé en ce qu'une solution de plasma est mise en contact avec au moins un support partiellement hydrophobe, échangeur d'ions ou non, et au moins un support hydrophile échangeur d'ions, les supports étant choisis indépendamment dans le groupe des matrices de polymères naturels polysaccharidiques, des matrices de polymères synthétiques, des oxydes minéraux revêtus d'un polymère polysaccharidique et des oxydes minéraux revêtus d'un film de polymère synthétique réticulé.

2. Procédé selon la revendication 1 caractérisé en ce que au moins un support hydrophile est échangeur de cations.

3. Procédé selon la revendication 1, caractérisé en ce que le support partiellement hydrophobe est échangeur d'anions et le support hydrophile est échangeur de cations.

4. Procédé selon la revendication 1 caractérisé en ce que la solution de plasma est mise en contact avec deux supports échangeurs d'anions dont l'un peut être hydrophile et l'autre est partiellement hydrophobe, et avec un support échangeur de cations hydrophile.

5. Procédé selon la revendication 4 dans lequel l'échangeur d'anions hydrophile précède l'échangeur d'anions partiellement hydrophobe.

6. Procédé selon l'une des revendications 1 à 5 caractérisé en ce que le support partiellement hydrophobe est choisi dans le groupe des polymères polysaccharidiques modifiés par fixation d'un radical hydrophobe, des adsorbants polymères synthétiques, des oxydes minéraux revêtus d'un polymère polysaccharidique modifié par fixation d'un radical hydrophobe et des oxydes minéraux revêtus d'un film de polymère réticulé hydrophobe.

7. Procédé selon l'une des revendications 1 à 5, caractérisé en ce que le support hydrophile est choisi dans le groupe des polymères polysaccharidiques, des polymères synthétiques hydrophiles insolubles dans l'eau, des oxydes minéraux revêtu d'un polymère polysaccharidique, des oxydes minéraux revêtus d'un film de polymère réticulé hydrophile.

8. Procédé selon l'une des revendications 1, 6 ou 7 caractérise en ce que l'oxyde minéral poreux présente une granulométrie de 4 µm à 5 mm, un diamètre poreux de 25 à 300 nm, une surface spécifique de 5 à 150 $m^2/g$.

9. Procédé selon la revendication 8 caractérisé en ce que l'oxyde minéral possède une granulométrie de 50 µm à 1 mm, un diamètre poreux de 60 à 150 nm et une surface spécifique de 20 à 50 $g/m^2$.

10. Procédé de fractionnement du plasma selon la revendication 1, par mise en contact avec au moins un échangeur d'anions et un échangeur de cations, caractérisé en ce que les échangeurs dont la capacité d'échange est inférieure à 2 m.eq/g sont constitués d'un support minéral poreux ayant une granulométrie de 4 µm à 5 mm, un diamètre poreux de 50 à 250 nm, une surface spécifique de 5 à 150 $m^2/g$ revêtu d'une quantité inférieure à 15 $mg/m^2$ d'un film de polymère réticulé hydrophobe contenant ou portant des groupes amines ou sels d'ammonium quaternaire pour au moins l'un des échangeurs et d'un film de polyvinyllactame réticulé portant des groupes acides carboxyliques pour l'autre échangeur.

11. Procédé selon la revendication 10, caractérisé en ce que la solution de plasma est mise en contact additionnellement, et de préférence initialement, avec un support minéral poreux de mêmes caractéristiques revêtu d'un polymère polysaccharidique portant des groupes amine ou sels d'ammonium quaternaire.

12. Procédé selon la revendication 10, dans lequel le polyvinyllactame qui revêt la surface du support échangeur de cations est obtenu par copolymérisation d'un vinyllactame et d'un acide carboxylique insaturé en présence d'un agent de réticulation polyfonctionnel.

13. Procédé selon la revendication 12, caractérisé en ce que le vinyllactame est le N-vinylpyrolidone.

14. Procédé selon la revendication 12, caractérisé en ce que l'acide carboxylique est l'acide acrylique.

15. Procédé selon l'une des revendications 12 à 14, caractérisé en ce que l'agent de réticulation est un dérivé silanique de formule $CH_2{=}CH - SiX_3$.

16. Procédé selon la revendication 10, caractérisé en ce que le polymère réticulé hydrophobe qui revêt la surface du support échangeur d'anions est obtenu à partir d'un monomère vinylaromatique.

17. Procédé selon l'une des revendications 1 à 16, caractérisé en ce que les quantités d'échangeurs d'anions sont comprises entre 250 et 2 000 ml par litre de plasma et les quantités d'échangeur de cations sont comprises entre 200 et 1 000 ml.

18. Procédé selon l'une des revendications 1 à 17, caractérisé en ce qu'il est réalisé en continu avec des séries de colonnes.

**Patentansprüche**

1. Verfahren zur Fraktionierung von Plasma durch Anionen- und Kationenaustauscher,
dadurch gekennzeichnet, daß man eine Plasmalösung mit zumindest einem teilweise hydrophoben Träger, der gegebenenfalls ein Ionenaustauscher ist, und zumindest einem ionenaustauschenden hydrophilen Träger in Berührung bringt, wobei die Träger unabhängig voneinander aus natürlichen Polysacchariden, synthetischen Polymeren, mineralischen Oxiden überzogen mit einem Polysaccharid oder einem Film eines synthetischen vernetzten Polymeren ausgewählt sind.

2. Verfahren nach Anspruch 1,
dadurch gekennzeichnet, daß zumindest ein hydrophiler Träger ein Kationenaustauscher ist.

3. Verfahren nach Anspruch 1,
dadurch gekennzeichnet, daß der teilweise hydrophobe Träger ein Anionenaustauscher und der hydrophile Träger ein Kationenaustauscher ist.

4. Verfahren nach Anspruch 1,
dadurch gekennzeichnet, daß man die Plasmalösung mit zwei Anionenaustauschern in Berührung bringt, von denen einer hydrophil und der andere zum Teil hydrophob sein kann, sowie mit einem hydrophilen Kationenaustauscher-Träger.

5. Verfahren nach Anspruch 4, wobei der hydrophile Anionenaustauscher dem teilweise hydrophoben Anionenaustauscher vorausgeht.

6. Verfahren nach einem der Ansprüche 1 bis 5,
dadurch gekennzeichnet, daß der teilweise hydrophobe Träger ausgewählt ist aus Polysacchariden, modifiziert mit Hilfe einer hydrophoben Gruppe, synthetischen polymeren Adsorptionsmitteln, mineralischen Oxiden, überzogen mit einem Polysaccharid und modifiziert mit einer hydrophoben Gruppe, oder mineralischen Oxiden, überzogen mit einem Film von hydrophoben vernetzten Polymeren.

7. Verfahren nach einem der Ansprüche 1 bis 5,
dadurch gekennzeichnet, daß der hydrophile Träger ausgewählt ist aus Polysacchariden, hydrophilen in Wasser unlöslichen synthetischen Polymeren, mineralischen Oxiden überzogen mit einem Polysaccharid und mineralischen Oxiden überzogen mit einem Film eines hydrophilen vernetzten Polymeren.

8. Verfahren nach einem der Ansprüche 1, 6 oder 7,
dadurch gekennzeichnet, daß das poröse mineralische Oxid eine Korngröße von 4 µm bis 5 mm, einen

Porendurchmesser von 25 bis 300 nm und eine spezifische Oberfläche von 5 bis 150 $m^2/g$ besitzt.

9. Verfahren nach Anspruch 8,
dadurch gekennzeichnet, daß das mineralische Oxid eine Korngröße von 50 µm bis 1 mm, einen Porendurchmesser von 60 bis 150 nm und eine spezifische Oberfläche von 20 bis 50 $m^2/g$ besitzt.

10. Verfahren zur Fraktionierung von Plasma nach Anspruch 1, indem zumindest ein Anionenaustauscher und ein Kationenaustauscher mit dem Plasma in Berührung gebracht wird,
dadurch gekennzeichnet, daß die Ionenaustauscher eine Austausch-Kapazität von < 2meq/g besitzen und aufgebaut sind aus einem porösen mineralischen Träger mit einer Korngröße von 4 µm bis 5 mm, einem Porendurchmesser von 50 bis 250 nm und einer spezifischen Oberfläche von 5 bis 150 $m^2/g$ und überzogen ist mit weniger als 15 $mg/m^2$ eines Films eines hydrophoben vernetzten Polymeren, enthaltend oder tragend Aminogruppen oder quaternäre Ammoniumsalze für zumindest einen der Ionenaustauscher und für den anderen einen Film aus einem vernetzten Polyvinyllactam mit Carbonsäuregruppen.

11. Verfahren nach Anspruch 10,
dadurch gekennzeichnet, daß die Plasmalösung weiters in Berührung gebracht wird - vorzugsweise anfangs - mit einem porösen mineralischen Träger gleicher Art, überzogen mit einem Polysaccharid, welches Amingruppen oder quaternäre Ammoniumsalze trägt.

12. Verfahren nach Anspruch 11, wobei das Polyvinyllactam auf der Oberfläche des Kationenaustauschers erhalten worden ist durch Copolymerisation von Vinyllactam und einer ungesättigten Carbonsäure in Gegenwart eines polyfunktionellen Vernetzungsmittels.

13. Verfahren nach Anspruch 12,
dadurch gekennzeichnet, daß das Vinyllactam N-Vinylpyrrolidon ist.

14. Verfahren nach Anspruch 12,
dadurch gekennzeichnet, daß die Carbonsäure Acrylsäure ist.

15. Verfahren nach einem der Ansprüche 12 bis 14,
dadurch gekennzeichnet, daß das Vernetzungsmittel ein Silan der Formel $CH_2=CH-SiX_3$ ist.

16. Verfahren nach Anspruch 10,
dadurch gekennzeichnet, daß das hydrophobe Vernetzungsmittel für die Oberfläche des Anionenaustauschers erhalten worden ist aus einer monomeren vinylaromatischen Verbindung.

17. Verfahren nach einem der Ansprüche 1 bis 16,
dadurch gekennzeichnet, daß die Mengen an Anionenaustauschern 250 bis 2 000 ml/l Plasma und die Mengen der Kationenaustauscher 200 bis 1 000 ml/l Plasma betragen.

18. Verfahren nach einem der Ansprüche 1 bis 17,
dadurch gekennzeichnet, daß es in einer Reihe von Kolonnen durchgeführt wird.

## Claims

1. A process for dividing up plasma by means of anionic and cationic ion exchangers characterised in that a solution of plasma is brought into contact with at least one partially hydrophobic carrier which may or may not be an ion exchanger, and at least one hydrophilic ion-exchanger carrier, the carriers being independently selected from the group of matrices of natural polysaccharidic polymers, matrices of synthetic polymers, mineral oxides coated with a polysaccharidic polymer and mineral oxides coated with a film of cross-linked synthetic polymer.

2. A process according to claim 1 characterised in that at least one hydrophilic carrier is a cation exchanger.

3. A process according to claim 1 characterised in that the partially hydrophobic carrier is an anion exchanger and the hydrophilic carrier is a cation exchanger.

4. A process according to claim 1 characterised in that solution of plasma is brought into contact with two anion-exchanger carriers of which one may be hydrophilic and the other is partially hydrophobic, and with a hydrophilic cation-exchanger carrier.

5. A process according to claim 4 wherein the hydrophilic anion exchanger precedes the partially hydrophobic anion exchanger.

6. A process according to one of claims 1 to 5 characterised in that the partially hydropbobic carrier is selected from the group of polysaccharidic polymers which are modified by fixing of a hydrophobic radical, synthetic polymer absorbents, mineral oxides coated with a polysaccharidic polymer modified by fixing of a hydrophobic radical and mineral oxides coated with a film of hydrophobic cross-linked polymer.

7. A process according to one of claims 1 to 5 characterised in that the hydrophilic carrier is selected from the group of polysaccharidic polymers, water-insoluble hydrophilic synthetic polymers, mineral oxides coated with a polysaccharidic polymer, and mineral oxides coated with a film of hydrophilic cross-linked polymer.

8. A process according to one of claims 1, 6 or 7 characterised in that the porous mineral oxide has a granulometry of 4 µm to 5 mm, a pore diameter of 25 to 300 nm and a specific surface area of 5 to 150 $m^2/g$.

9. A process according to claim 8 characterised in that the mineral oxide has a granulometry of 50 µm to 1 mm, a pore diameter of 60 to 150 nm and a specific surface area of 20 to 50 $m^2/g$.

10. A process for dividing up plasma according to claim 1 by bringing it into contact with at least one anion exchanger and one cationic exchanger characterised in that the exchangers whose exchange capacity is lower

that 2 m.eq/g are formed by a porous mineral carrier having a granulometry of 4 µm to 5 mm, a pore diameter of 50 to 250 nm, a specific surface area of 5 to 150 $m^2/g$, coated with an amount of less than 15 $mg/m^2$ of a film of hydrophobic cross-linked polymer containing or bearing amino groups or quaternary ammonium salts for at least one of the exchangers and a film of cross-linked polyvinyllactame bearing carboxylic acid groups for the other exchanger.

11. A process according to claim 10 characterised in that the solution of plasma is additionally brought into contact, preferably initially, with a porous mineral carrier having the same characteristics, coated with a polysaccharidic polymer bearing amino groups or quaternary ammonium salts.

12. A process according to claim 10 wherein the polyvinyl lactame which coats the surface of the cation-exchanger carrier is produced by co-polymerisation of a vinyl lactame and an unsaturated carboxylic acid in the presence of a polyfunctional cross-linking agent.

13. A process according to claim 12 characterised in that the vinyl lactame is N-vinylpyrrolidone.

14. A process according to claim 12 characterised in that the carboxylic acid is acrylic acid.

15. A process acccrding to one of claims 12 to 14 characterised in that the cross-linking agent is a silanic derivative of the formula $CH_2 = CH - SiX_3$.

16. A process according to claim 10 characterised in that the hydrophobic cross-linked polymer which coats the surface of the anionexchanger carrier is produced from a vinylaromatic monomer.

17. A process according to one of claims 1 to 16 characterised in that the amounts of anion exchangers are between 250 and 2000 ml per litre of plasma and the amounts of cation exchanger are between 200 and 1000 ml.

18. A process according to one of claims 1 to 17 characterised in that it is carried out continuously with series of columns.